# EUROPEAN PATENT SPECIFICATION

(11) **EP 1 177 729 B1**
(45) Date of publication and mention of the grant of the patent: **23.01.2008**
(21) Application number: 01306414.2
(22) Date of filing: 26.07.2001
(51) Int. Cl.: A23L 1/30, A23L 1/23, A61P 3/06

(54) **Pharmaceutical and food compositions containing "wood alcohols" or "wood sterols" useful for lowering serum cholesterol**
Pharmazeutische- und Nahrungsmittelzusammenzetzungen mit "Holzalkohol" und "Holzsterol" zur Senkung des Serumcholesterins
Compositions pharmaceutiques et alimentaires contenant des "alcools de bois" et "stérols de bois" pour diminuer le cholesterol sérique

(30) Priority: 03.08.2000 CL 20002187
(43) Date of publication of application: 06.02.2002
(73) Proprietor: Härting, Thomas Francis, Quilicura (CL)
(72) Inventor: Markovits Schersl, Endre, Quilicura (CL); Markovits Rojas, Alejandro, Quilicura (CL); Fuenzalida Diaz, Miguel, Quilicura (CL)
(74) Representative: Crouch, David John

(56) References cited:
- WO-A-01/32031
- US-A- 4 391 732
- US-A- 5 725 803
- US-A- 5 952 393
- PATENT ABSTRACTS OF JAPAN vol. 011, no. 315 (C-451), 14 October 1987 (1987-10-14) & JP 62 099323 A (YOSHIHIDE HAGIWARA), 8 May 1987 (1987-05-08)

## Description

The present invention is related to food and pharmaceutical compositions containing fatty alcohols of 26 or less carbon atoms per molecule such as octadecanol, eicosanol, docosanol, tetracosanol or hexacosanol and or wood sterols useful for treating hypercholesteromia in human beings.

Disorders of lipid metabolism especially harmful effects caused by high levels of serum cholesterol have been intensively investigated.

Cholesterol levels in blood over 200 mg/dl constitute the main risk factor of coronary diseases, the most frequent cause of death, principally in developed countries. However, the risk factor is not only related to high cholesterol level in blood, but also to different forms of total cholesterol. A high level of low-density lipoprotein or LDL cholesterol and very low-density lipoprotein or VLDL cholesterol in blood constitutes a problem because these lipoproteins are very likely to remain in the cardiovascular system causing the formation of plaques in the coronary arteries. Likewise, low levels of high-density lipoproteins or HDL cholesterol constitute an additional risk factor because they are useful in removing the form of cholesterol that blocks arteries. Therefore total cholesterol level and total cholesterol HDL cholesterol ratio must be considered for evaluating the risk of coronary diseases.

Many food and pharmaceutical compositions containing natural products or by-products that lower serum cholesterol are used for treating hypercholesterolemia. Among natural products recently disclosed for this end are the long chain aliphatic alcohols or fatty alcohols generally denominated policosanols. This fatty alcohols is found in vegetal waxes where they occur esterified with fatty acids. During the processing of vegetal matter, waxes are normally hydrolyzed which leads to mixtures comprising different fatty alcohols. The aliphatic chain of these alcohols may contain from 18 to 38 carbon atoms. It is thought that policosanols interfere with cholesterol synthesis in the liver.

US Patent No. 5,856,316 discloses a composition of policosanols useful for treating hypercholesterolemia and other metabolic disorders containing a mixture of aliphatic alcohols having from 24 to 34 carbon atoms per molecule which are obtained from sugarcane waxes.

US Patent No. 5,952,393 discloses a composition comprising mixtures of phytosterols and policosanols useful for lowering serum cholesterol. Phytosterols of the invention consist of a mixture of β-sitosterol, campesterol and stigmasterol obtained from vegetal oil, meanwhile the policosanols, which comprise aliphatic alcohols having from 20 to 36 carbon atoms per molecule, derive from rice bran wax.

Japanese Patent Publication No. 62099323 discloses the use of hexacosanol as a useful cholesterol lowering agent.

US Patent No. 5,725,803 discloses blends of fatty alcohols and free phytosterols, and the use thereof in cosmetic and pharmaceutical formulations.

WO 01/32031 A2 discloses a composition comprising a mixture of phytosterol and/or phytosterol ester and a surfactant(s). This document refers therein to studies concerning the use of beta-sitosterol to produce reductions in the amount of cholesterol in the blood (Farquhar, J.W. et al., Circulation, 14, 77-82(1956)), US Patent No. 5,244,887 which is directed to plant stanol food additives for reducing the absorption of cholesterol in the gastro-intestinal tract, and EP 0 897 671 which discloses aqueous dispersions of high melting lipids, such as plant sterols, with non-sterol emulsifiers.

Table I shows composition and content of mixtures of policosanols disclosed in the sate of art quoted, along with compositions of policosanols of the present invention also called "wood alcohols". Table I compares the fatty alcohol composition of sugarcane wax, rice bran wax disclosed in the state of art and that of "wood sterols" contained in either tall oil pitch or tall oil soap of the present invention.

**Table I: Fatty alcohol or policosanol composition of sugarcane wax, rice bran wax and "wood alcohols"**

| Source | Wax alcohols | | "Wood alcohols" | |
|---|---|---|---|---|
| Aliphatic alcohol | Sugarcane wax | Rice bran wax | Tall oil pitch | Tall oil soap |
| Octadecanol (C18) | -- | -- | -- | 1.0 - 10.0 |
| Eicosanol (20) | -- | -- | 1.0 - 5.0 | 5.0 - 25.0 |
| Docosanol (22) | -- | 1.1 - 1.6 | 5.0 - 30.0 | 2.0 - 60.0 |
| Tetracosanol (24) | 0.5 - 1.0 | 9.7 - 14.0 | 20.0 - 60.0 | 20.0 - 50.0 |
| Hexacosanol (26) | 5.5 - 8.5 | 8.9 - 12.7 | 15.0 - 50.0 | 1.0 - 5.0 |
| Heptacosanol (27) | 2.0 - 3.5 | -- | -- | -- |
| Octacosanol (28) | 60.0 - 70.0 | 16.9 - 24.3 | -- | -- |
| Nonacosanol (29) | 0.4 - 1.2 | -- | -- | -- |
| Triacontanol (30) | 10.0 - 15.0 | 25.3 - 36.3 | -- | -- |
| Dotriacontanol (32) | 4.0 - 6.0 | 14. 1 - 20.2 | -- | -- |
| Tetratriacontanol (34) | 0.4 - 2.0 | 6:7 - 9.6 | -- | -- |
| Hexatriacontanol (36) | -- | 1.5 - 2.2 | -- | -- |

It is noticed that policosanols from sugarcane wax do not contain aliphatic alcohols with less than 24 carbon atoms per molecule and the alcohols with less than 24 carbon atoms per molecule made up less than 2% of total alcohol in policosanols of rice bran wax. In both compositions, fatty alcohols with 28 or more carbon atoms per molecule constitute over 70% of the total fatty alcohols, and that "wood alcohols" do not contain fatty alcohols with more than 26 carbon atoms per molecule.

On the basis of compositions of policosanols disclosed in the state of art for their serum cholestrol lowering effect, it might be expected that said effect of policosanols would be characterisitic only of aliphatic alcohols having more than 26 carbon atoms per molecule. However, it has been surprisingly discovered that "wood alcohols"comprising octadecanol (estearyl alcohol), eicosanol (arachidyl alcohol), docosanol (behenyl alcohol), tetracosanol (lignoceryl alcohol) and hexacosanol (ceryl alcohol), the "wood alcohols" of the present invention exhibit an important serum cholesterol lowering effect when are ingested orally in low dose either in food or pharmacological compositions. Mixtures of octadecanol, eicosanol, docosanol, tetracosanol or hexacosanol of the present invention having a mean composition shown in Table I are by-products of cellulose industry or by-products of tall oil distillation hereafter called "wood alcohols". These "wood alcohols" can be produced in high yield and purity by processes disclosed in Chilean patent applications 85/98 and 2026/99 and reproduced in Examples 1 and 2.

Free sterols or steryl esters are also efficient serum cholesterol lowering agents. It has been observed that when a mixture of "wood alcohols" with "wood sterols" or esterified "wood sterols" are used in food or pharmaceutical compositions, the hypocholesterolemic effect of the mixture is higher that the effect of either "wood alcohols" or "wood sterols" alone especially in lowering LDL cholesterol; that is there is surprising synergestic effect of the mixture on the reduction of the ratio total cholesterol/high density lipoprotein (HDL).

Processes for the production of "wood sterols" are disclosed as well in Chilean patent application 2026/99.

Therefore, an objective of the present invention is to provide food and pharmaceutical compositions for lowering serum cholesterol preferably in human beings, where the composition comprise long chain aliphatic alcohols having 26 or less carbon atoms per molecule.

A further objective of the present invention is to provide a method for lowering serum cholesterol preferably in human beings, by means of orally administering food or pharmaceutical compositions comprising long chain aliphatic alcohols having 26 or less carbon atoms per molecule. Daily dose of said compositions comprise 0.01 and 50 mg of wood alcohols" per kilogram of body weight.

Food and pharmaceutical compositions useful for lowering serum cholesterol comprise "wood alcohols" or "wood alcohols" and either free or esterified "wood sterols". "Wood alcohols" can be produced according to processes disclosed in Chilean patent application N°85/98. "Wood sterols" comprising both sterols and stanols can be obtained according to processes disclosed in Chilean patent application N°2026/99.

In the present invention, the term "wood alcohols" stand for a mixture comprising octadecanol, eicosanol, docosanol, tetracosanol or hexacosanol. The term "wood sterols" stands for a mixture comprising saturated sterols or stanols and non-saturated sterols. Table II shows the average composition of "wood sterols" Likewise, the term "esterified wood sterols" refers to a mixture containing saturated or non-saturated sterols and esterified with fatty acids. The esterified wood sterols can be obtained by processes disclosed in Chilean patent application N°. 209/00.

**Table II: Average composition of "wood sterols"**

| **Sterol** | **% in weight** |
|---|---|
| Beta-sitosterol | 45-70 |
| Beta-sitostanol | 10-25 |
| Campesterol | 3-15 |
| Campestanol | 1-10 |
| Stigmasterol | Less than 5 |

Food composition useful for lowering blood serum levels of cholesterol can be prepared by incorporating "wood alcohols" into fatty foods such as edible oil, butter, margarine, chocolate, milk or its by-products (ice cream, yogurt, cheese) or by incorporating "wood alcohols" into non fatty foods.

Ice cream containing "wood alcohols" or "wood sterols" or esterified "wood sterols" can be prepared according to procedures disclosed in the state of the art. Said alcohols or sterols can partly substitute the fatty matter employed in the elaboration of ice cream.

Pharmaceutical compositions useful for lowering serum cholesterol can be prepared by formulating compositions comprising "wood alcohols" or "wood alcohols" and "wood sterols" or esterified "wood sterols" as active components toghether with pharmaceutically acceptable vehicles such as excipients, binders, stabilizers, lubricants, preservatives or coating agents.

The present invention is directed to the use of a mixture as set out in the accompanying claims 1, 2, 4 or 5. Preferred features are set out in the sub-ordinate claims 3, 6 and 7.

According to the present invention, pharmaceutical compositions can be provided in the form of tablets, capsules, pills, syrup, suppositories, subcutaneous dispensers or dermal patches.

### Examples:

### Example 1: "Wood alcohols" from tall oil soap [outside the scope of the invention]

150 g of unsaponifiable matter of tall oil soap with 0.8% humidity was fed at the rate of 2 g/min into a shorth path distillation column, model KDL-4 UIC GmbH. The evaporator temperature was kept at 210°C, the temperature of the internal condenser was 65°C, and the pressure in the column was 0.2 mbar. 63.2 g of distillate or overhead product and 82.8 g of bottom product were recovered. Then, 50 g of the distillate was dissolved in 500 g of methanol at 50°C. Upon cooling down de dissolution to 15 °C a precipatate was formed and this precipate was recovered by filtration and washed with fresh methanol. The yield was 2.8 g of precipitate.
The total fatty alcohol content of the precipitate was 95 % in weight and its composition is shown in Table III.

**Table III: Composition of the precipate of Example 1**

| **Fatty alcohol** | **% in weight** |
|---|---|
| Octadecanol | 4.0 |
| Eicosanol | 23.4 |
| Docosanol | 27.7 |
| Tetracosanol | 34.8 |
| Hexacosanol | 5.1 |
| Heptacosanol | -- |
| Octacosanol | -- |
| Nonacosanol | -- |
| Triacosanol | -- |
| Dotriacontanol | -- |
| Tetratriacontanol | -- |
| Hexatriacontanol | -- |

The mixture of policosanols shown in Table III is an example of "wood alcohols" derived from black liquor soaps.

### Example 2: "Wood alcohols" from tall oil pitch

A precipitate fom unsaponifiable matter from tall oil pitch was obtained as described in Example 1. The composition of the mixture containing 97.4 % in weigth of policosanols is shown in Table IV.

**Table IV: Composition of the precipitate o Example 2**

| **Fatty alcohol** | **% in weight** |
|---|---|
| Octadecanol | -- |
| Eicosanol | 2.5 |
| Docosanol | 19.7 |
| Tetracosanol | 53.1 |
| Hexacosanol | 22.1 |
| Heptacosanol | -- |
| Octacosanol | -- |
| Nonacosanol | -- |
| Triacosanol | -- |
| Dotriacontanol | -- |
| Tetratriacontanol | -- |
| Hexatriacontanol | -- |

The mixture of policosanols shown in Table IV is an example of "wood alcohols" derived from tall oil pitch.

"Wood alcohols" as shown in the previous examples, typically do not contain fatty alcohols or policosanols with more than 26 carbon atoms per molecule of fatty alcohol.

### Example 3 : "Wood sterols" from tall oil pitch

200 g of bottom product obtained by distilling unsaponifiable matter of tall oil pitch as described in Example 1 were dissolved at 60°C in 700 g of a hepthanol/methanol/water mixture (3/0.1/0.1 v/v) and then cooled down to 20°C until a precipitate was formed which was filtered out, washed with methanol and dried. The composition of the precipitate is shown in Table V.

**Table V: Composition of the precipitate of Example 3**

| **Sterol** | **% in weight** |
|---|---|
| Beta-sitosterol | 68.4 |
| Beta-sitostanol | 21.8 |
| Campsterol | 6.1 |
| Campestanol | 1.1 |
| Stigmasterol | Less than 1 |

The mixture of sterols shown in Table V is an example of "wood sterols" derived from tall oil pitch.

### Example 4: Preparation of mayonnaise containing "wood alcohols" and esterified "wood sterols".

"Wood sterols" used in this example had the composition shown in Table V and "wood alcohols" used in this example had the composition shown in Table IV. "Wood sterols" were esterified with ethyl esters fatty acids of raps oil at 80°C and 1 mbar in presence of 0.5% of sodium ethylate as described in Chilean patent application N°209/00. The composition of the mayonnaise is shown in Table VI.

**Table VI: Composition of mayonnaise of Example 4**

| **Ingredient** | **% in weight** |
|---|---|
| Modified starch | 2.86 |
| Methylhydroxicellulose | 1.18 |
| Salt | 0.75 |
| Powdered mustard | 0.62 |
| Saccharine | 0.19 |
| Clove, garlic. onion, pepper | 0.93 |
| Anhydrous citric acid | 0.50 |
| Sodium citrate | 0.16 |
| Ascorbic acid | 0.09 |
| Sorbitol | 0.75 |
| Vinegar | 2.00 |
| Egg yolk | 7.45 |
| Vegetal oil | 20.00 |
| Esterified "wood sterols" | 5.00 |
| "Wood alcohols" | 0.10 |
| Water | 57.42 |

### Preparation:

"Wood sterols" used in this example had the composition shown in Table V and
"wood alcohols" used in this example had the composition shown in Table IV.

100 g of mayonnaise are prepared by mixing in a beaker water with the ingredients shown on the table excepting vegetal oil, egg yolk, esterified "wood sterols" and "wood alcohols", to form an aqueous solution. In a separate beaker 20 g of vegetal oil were heated to 80°C and "wood alcohols" were added to form a first homogeneous mixture. Next, esterified "wood sterols" were gradually added to the mixture to form a second homogeneous mixture which was let to cool to 40°C and then the egg yolk was added with mixing to form a third mixture. After reaching said third mixture room temperature, the aqueous solution was added to it gradually under homogeneizing conditions. A daily intake by an adult of about 20 of the maryonnaise of the example should be sufficient for significantly lowering blood serum cholesterol level.

### Example 5 . Margarine containing "wood alcohols".

250 g of table margarine were melted and heated to approximately 80°C, then 200 mg of -wood alcohols" of the compostion shown in Table IV are were mixed in forming a homogeneous mixture. When the mixture was cooled down to room temperature it formed a solid and readily spreadable product whose organoleptic characteristics do not differ from conventional margarine. The described process can be applied to any animator vegetal fatty matter used as table product, e.g. butter, or as a baking product. A daily intake by an adult of about 20 of the margarine of the example should be sufficient for significantly lowering blood serum cholesterol level.

### Example 6: Ice cream containing "wood alcohols" and "wood sterols" as non milk fats.

Ice cream of the composition shown in Table VII was prepared

**Table VII. Composition of ice cream of Example 6**

| **Ingredient** | **% in weight** |
|---|---|
| Water | 62.00 |
| Lactose | 6.00 |
| Sucrose | 12.00 |
| Sodium caseinate | 0.70 |
| "Wood alcohols" | 0.02 |
| "Wood sterols" | 2.00 |
| Hydrogenated vegetable oil | 11.00 |
| Powdered skimmed milk | 4.90 |
| Mono and diglycerides | 0.50 |
| Mineral salts | 0.80 |
| Colorant and flavoring agents | 0.08 |

### Preparation.

Lactose, sucrose, skimmed milk, mineral salts and sodium caseinate were dissolved at about 60°C in a beaker. In another beaker hydrogenated vegetable oil was melted and mixed with the "wood alcohols", "wood sterols" and mono and diglycerides. "Wood alcohols" and "wood sterols" had the same composition as those used in Example 4. Then, the content of the two beakers were vigorously mixed together at about 80 °C and the emulsion so formed were homogenized using an Eberbach 8017 laboratory homogenizer. The resulting mixture was whipped with air and then frozen. Ice cream density was about 620 g per liter and its appearance and organoleptic characteristics did not differ significantly from ice cream similarly prepared containing 12% of fatty matter but lacking '"wood alcohols" or "wood sterols". A daily intake by an adult of about 50 g of the ice cream of the example should be sufficient for significantly lowering blood serum cholesterol level.

### Example 7: Preparation of cooking oil containing "wood alcohols" and "wood sterols"

97.4 g of sunflower oil were charged into a 250 ml flat bottom glass flask with 4 standar ground necks provided high shear mixer, nitrogen bleaching, followed by the gradual addition of 100 mg of "wood alcohols" and 2.5 g of "wood sterols" of the same composition as those used in Example 4 until a homogeneous mixture was obtained, which remained liquid at room temperature. Said mixture can be used as conventional cooking or salad dressig oil. A daily intake by an adult of about 20 g of oil of the example should be sufficient for significantly lowering blood serum cholesterol level.

### Example 8: Reconstituted milk containing "wood alcohols".

1.6 g of sodium salt of raps oil fatty acids are dissolved in 1 liter of skimmed milk. Next, 30.4 g of cooking oil prepared as described in example 7 but containing 1 % in weight of "wood alcohols" are added dropwise to the milk under vigorous agitation using a high shear mixer. After addition of the oil, stirring is continued for 15 minutes more. At the end a reconstituted stable milk is obtained. A daily intake by an adult of about 100 ml of reconstituted milk of the example should be sufficient for significantly lowering blood serum cholesterol level.

### Example 9: Beverage containing wood alcohols and esterified wood sterols.

Mixture C containig 92.5 g of raps oil, 500 mg of "wood alcohols" and 7 g of "wood sterols" is prepared as described in Example 4. Next, mixtures A and B are prepared.

### Mixture A

| | |
|---|---|
| Tap water | 700.0 g |
| High fructose syrup | 80.0 g |
| Sucrose | 20.0 g |
| Citric acid | 20.0 g |
| Sodium citrate | 10.0 g |
| Sodium benzoate | 4.9 g |
| Sodium sorbate | 5.0 g |
| Aspartame | 0.1 g |

### Mixture B

| | |
|---|---|
| Tap water | 95.0 g |
| Guar gum | 31.6 g |
| Titanium dioxide | 0.5 g |
| Caramel (as colorant) | 1.5 g |
| Tartrazine | 1.0 g |
| Mixture C | 30.4 g |

Components of mixture B are vigorously mixed together to form an emulsion. Then mixture B is poured gradually and under vigorous stirring over 2 l of a mixture A, until reaching complete homogeneity. A slightly turbid beverage is obtained. A daily intake by an adult of about 200 ml of beverage of the example should be sufficient for significantly lowering blood serum cholesterol level.

### Example 10: Preparation of tablets containing "wood alcohols"

20 g of "wood alcohols" of the composition shown in Table IV are melted at approximately 55°C and vigorously mixed with 386 g of calcium phosphate used as filler. Once the mixture is cooled down at room temperature, it is thoroughly mixed with 24 g of estearic acid and 7 g of magnesium estearate as lubricants, 10 g of silicon dioxide as diluent and 50 g of cellulose as coating material. The amount of material prepared is used to make 1,000 tablets by compressing the resulting mixture in a conventional pill press. A daily intake by an adult of up to 4 tablets of the example should be sufficient for significantly lowering blood serum cholesterol level.

### Example 11: Tablets containing "wood alcohols" and "wood sterols"

A composition of tablets which is made as described in Example 10 is the following:

| **Component** | **mg/tablet** |
|---|---|
| "Wood alcohols" | 20 |
| "Esterified wood sterols" | 480 |
| Lactose | 400 |
| Calcium lactate | 100 |
| Magnesium estearate | 10 |
| Talc | 10 |
| Sucrose. | 10 |
| Microcristalline cellulose | 70 |

"Wood sterols" used in this example had the composition shown in Table V and "wood alcohols" used in this example had the composition shown in Table IV. A daily intake by an adult of up to 4 tablets of the example should be sufficient for significantly lowering blood serum cholesterol level.

### Example 12: Effect of "wood alcohols" and estrified "wood sterols" in test animals

"Wood sterols" used in this example had the composition shown in Table V and "wood alcohols" used in this example had the composition shown in Table IV 30 Sprague Dawley male rats divided at random into five groups of six animals each are fed for ten days with the following diets:

| **Diet** | **Ingredients** |
|---|---|
| C₀ | P |
| C₁ | P + C |
| C₂ | P + C + EE |
| C₃ | P + C + PC |
| C₄ | P + C + PC + EE |

| | |
|---|---|
| where P : *Champion* pellets ground and mixed with 5% in weight of corn oil C : 1% in weight of cholesterol Merck, 95 % purity EE : 1% in weight of esterified "wood sterols" PC : 1% in weight of "wood alcohols". | |

Percentages are relative to the mixture of ingredients.
Dietary treatment was individually applied and corporal weight and dietary consumption were measured. At the end of the feeding period, total cholesterol content (TC) in the liver and serum and LDL cholesterol in serum of each animal were measured. The following results were obtained.

| **Food** | **TC liver** **(mg/g)** | **Sample standard deviation** | **TC serum** **(mg/dl)** | **Sample standard deviation** | **LDL serum** **(mg/dl)** | **Sample standard deviation** |
|---|---|---|---|---|---|---|
| C₀ | 1.53 | 0.12 | 80.40 | 7.13 | 56.72 | 5.09 |
| C₁ | 2.82 | 0.19 | 96.80 | 7.80 | 71.26 | 8.34 |
| C₂ | 1.28 | 0.15 | 87.58 | 1.14 | 71.75 | 9.35 |
| C₃ | 2.90 | 0.10 | 82.17 | 4.30 | 48.03 | 3.88 |
| C₄ | 1.20 | 0.11 | 72.30 | 4.27 | 38.72 | 4.43 |

### Statistics

Comparisons of pair of means were made by Duncan multiple range test and the results were:

### Total cholesterol in liver:

| **Difference** | **Significance at 5%** |
|---|---|
| C₃ - C₄ | Significant |
| C₃ - C₂ | Significant |
| C₃ - C₀ | Significant |
| C₃ - C₁ | Non-significant |
| C₁ - C₄ | Significant |
| C₁ - C₂ | Significant |
| C₁ - C₀ | Significant |
| C₀ - C₄ | Significant |
| C₀ - C₂ | Significant |
| C₂ - C₄ | Non-significant |

### Total cholesterol in serum:

| **Difference** | **Significance at 5%** |
|---|---|
| C₁ - C₄ | Significant |
| C₁ - C₀ | Significant |
| C₁ - C₃ | Significant |
| C₁ - C₂ | Significant |
| C₂ - C₄ | Significant |
| C₂-C₀ | Non-significant |
| C₂ - C₃ | Non-significant |
| C₃ - C₄ | Significant |
| C₃ - C₀ | Non-significant |
| C₀ - C₄ | Non-significant |

### LDL cholesterol in serum:

| **Difference** | **Significance at 5%** |
|---|---|
| C₂ - C₄ | Significant |
| C₂ - C₃ | Significant |
| C₂ - C₀ | Significant |
| C₂ - C₁ | Significant |
| C₁ - C₄ | Non-significant |
| C₁ - C₃ | Non-significant |
| C₁ - C₀ | Non-significant |
| C₀ - C₄ | Significant |
| C₀ - C₃ | Significant |
| C₃ - C₄ | Significant |

Analysis of results indicates that a diet rich in cholesterol increases content of total cholesterol in liver, blood serum as well as LDL level in serum.

Presence of policosanols in diet does not compensate said increase in the liver but presence of esterified "wood sterols" does. It can be observed that decrease of cholesterol in the liver is lower with food C₄ than with food C₂, although this difference is not significant. It can also be observed that there seems to be no correlation between cholesterol content in the liver and content of total cholesterol and LDL cholesterol in serum.

As expected, a diet rich in cholesterol increases total cholesterol in serum which is compensated by the presence of "wood alcohols" or esterified "wood sterols " in the diet. Likewise, the presence of one of these compounds in diet does not cause a Significant difference in serum cholesterol with respect to a diet without supplementary cholesterol. However, it can be observed that simultaneous presence of "wood alcohols" and esterified "wood sterols" in a diet rich in cholesterol decrease significantly cholesterol levels with respect to diet without supplementary cholesterol evidencing some kind of sinergism in their cholesterol lowering activities between both.
Effects of "wood alcohols" in decreasing serum LDL cholesterol are significant and it can be observed again a synergestic effect of "wood alcohols" and esterified "wood sterols" in the diet.

### Example 13: Tests in human subjects

In a non-controlled experience, five male adults consumed margarine enriched with "wood alcohol"s prepared as described in Example for six weeks. Portions of margarine daily consumed amounted to a daily intake between 20 and 30 mg of "wood alcohols". Lipid profiles, total cholesterol (TC), LDL cholesterol and HDL cholesterol (mg/dl) were the folowing:

| **Subject** | **Initial TC** | **Final TC** | **Initial LDL** | **Final LDL** | **Initial HDL** | **Final HDL** |
|---|---|---|---|---|---|---|
| 1 | 234 | 207 | 141 | 115 | 62 | 61 |
| 2 | 215 | 185 | 132 | 105 | 55 | 52 |
| 3 | 196 | 181 | 128 | 114 | 42 | 44 |
| 4 | 202 | 203 | 130 | 129 | 40 | 41 |
| 5 | 184 | 161 | 115 | 99 | 48 | 45 |
| Average | 206 | 187 | 129 | 112 | 49 | 48 |

Student's test for the paiwise comparison ot the means indicate a significant decrease of total cholesterol with a confidence level of 95% and a significant decrease of LDL cholesterol with a confidence level of 90%.

## Claims

1. The use of a mixture of higher primary aliphatic alcohols isolated from tall oil soap for the manufacture of a food formulation for the reduction of serum cholesterol levels in human subjects, **characterised in that** the mixture of higher primary aliphatic alcohols is included in the food formulation and comprises the following quantitative composition in weight percentage:
| | |
|---|---|
| octadecanol | 1.0 -10.0 |
| eicosanol | 5.0 - 25.0 |
| docosanol | 2.0 - 60.0 |
| tetracosanol | 20.0 - 50.0 |
| hexacosanol | 1.0 - 5.0. |

2. The use of a mixture of higher primary aliphatic alcohols isolated from tall oil pitch for the manufacture of a food formulation for the reduction of serum cholesterol levels in human subjects, **characterised in that** the mixture of higher primary aliphatic alcohols is included in the food formulation and comprises the following quantitative composition in weight percentage:
| | |
|---|---|
| eicosanol | 1.0 - 5.0 |
| docosanol | 5.0 - 30.0 |
| tetracosanol | 20.0 - 60.0 |
| hexacosanol | 15.0 - 50.0. |

3. The food formulation of claim 1 or claim 2 **characterised in that** it further comprises table margarine, butter, mayonnaise, edible oil, milk, chocolate, cheese, ice cream or yogurt.

4. The use of a mixture of higher primary aliphatic alcohols isolated from tall oil soap for the manufacture of a medicament for the reduction of serum cholesterol levels in human subjects, **characterised in that** the mixture of higher primary aliphatic alcohols is included in the medicament and comprises the following quantitative composition in weight percentage:
| | |
|---|---|
| octadecanol | 1.0 - 10.0 |
| eicosanol | 5.0 - 25.0 |
| docosanol | 2.0 - 60.0 |
| tetracosanol | 20.0 - 50.0 |
| hexacosanol | 1.0 - 5.0. |

5. The use of a mixture of higher primary aliphatic alcohols isolated from tall oil pitch for the manufacture of a medicament for the reduction of serum cholesterol levels in human subjects, **characterised in that** the mixture of higher primary aliphatic alcohols is included in the medicament and comprises the following quantitative composition in weight percentage:
| | |
|---|---|
| eicosanol | 1.0- 5.0 |
| docosanol | 5.0 - 30.0 |
| tetracosanol | 20.0- 60.0 |
| hexacosanol | 15.0- 50.0. |

6. The use according to claim 4 or claim 5 **characterised in that** the medicament further comprises a pharmaceutically acceptable excipient, binder, stabilizer, lubricant, preservative or coating agent.

7. The use according to claim 6 **characterised in that** the medicament is in the form tablets, capsules, pills or syrup.

## Patentansprüche

1. Die Verwendung einer Mischung höherer primärer aliphatischer Alkohole, gewonnen aus Tallölseife, zur Herstellung einer Nahrungsmittelzusammensetzung zur Verringerung der Cholesterinspiegel beim Menschen, **dadurch gekennzeichnet, dass** die Mischung höherer primärer aliphatischer Alkohole in der Nahrungsmittelzusammensetzung eingeschlossen ist und die folgende quantitative Zusammensetzung in Gewichtsprozent aufweist:
| | |
|---|---|
| Octadecanol | 1,0 - 10,0 |
| Eicosanol | 5,0 - 25,0 |
| Docosanol | 2,0 - 60,0 |
| Tetracosanol | 20,0 - 50,0 |
| Hexacosanol | 1,0 - 5,0. |

2. Die Verwendung einer Mischung höherer primärer alphatischer Alkohole, gewonnen aus Tallölpech, zur Herstellung einer Nahrungsmittelzusammensetzung zur Verringerung der Cholesterinspiegel beim Menschen, **dadurch gekennzeichnet, dass** die Mischung höherer primärer alphatischer Alkohole in der Nahrungsmittelzusammensetzung eingeschlossen ist und die folgende quantitative Zusammensetzung in Gewichtsprozent aufweist:
| | |
|---|---|
| Eicosanol | 1,0 - 5,0 |
| Docosanol | 5,0 - 30,0 |
| Tetracosanol | 20,0 - 60,0 |
| Hexacosanol | 15,0 - 50,0. |

3. Nahrungsmittelzusammensetzung nach Anspruch 1 oder Anspruch 2, **dadurch gekennzeichnet, dass** sie ferner Speisemargarine, Butter, Majonaise, Speiseöl, Milch, Schokolade, Käse, Eiscreme oder Joghurt enthält.

4. Verwendung einer Mischung höherer primärer aliphatischer Alkohole, gewonnen aus Tallölseife, zur Herstellung eines Medikaments zur Verringerung der Cholesterinspiegel beim Menschen, **dadurch gekennzeichnet, dass** die Mischung höherer primärer aliphatischer Alkohole in dem Medikament eingeschlossen ist und die folgende quantitative Zusammensetzung in Gewichtsprozent aufweist:
| | |
|---|---|
| Octadecanol | 1,0 - 10,0 |
| Eicosanol | 5,0 - 25,0 |
| Docosanol | 2,0 - 60,0 |
| Tetracosanol | 20,0 - 50,0 |
| Hexacosanol | 1,0 - 5,0. |

5. Verwendung einer Mischung höherer primärer aliphatischer Alkohole, gewonnen aus Tallölpech, zur Herstellung eines Medikaments zur Verringerung der Cholesterinspiegel beim Menschen, **dadurch gekennzeichnet, dass** die Mischung höherer primärer aliphatischer Alkohole in dem Medikament eingeschlossen ist und die folgende quantitative Zusammensetzung in Gewichtsprozent aufweist:
| | |
|---|---|
| Eicosanol | 1,0 - 5,0 |
| Docosanol | 5,0 - 30,0 |
| Tetracosanol | 20,0 - 60,0 |
| Hexacosanol | 15,0 - 50,0. |

6. Die Verwendung nach Anspruch 4 oder Anspruch 5, **dadurch gekennzeichnet, dass** das Medikament ferner einen pharmazeutisch annehmbaren Träger, Bindemittel, Stabilisator, Schmiermittel, Konservierungsmittel oder Überzugsmittel umfasst.

7. Verwendung nach Anspruch 6, **dadurch gekennzeichnet, dass** das Medikament in Form von Tabletten, Kapseln, Pillen oder Sirup vorliegt.

## Revendications

1. Utilisation d'un mélange d'alcools aliphatiques primaires supérieurs isolé de savon de tallöl pour la fabrication d'une formulation alimentaire destinée à la réduction des taux de cholestérol sérique chez des sujets humains, **caractérisée en ce que** le mélange d'alcools aliphatiques primaires supérieurs est inclus dans la formulation alimentaire et comprend la composition quantitative suivante en pourcentages en poids :
| | |
|---|---|
| octadécanol | 1,0 - 10,0 |
| eicosanol | 5,0 - 25,0 |
| docosanol | 2,0 - 60,0 |
| tétracosanol | 20,0 - 50,0 |
| hexacosanol | 1,0 - 5,0. |

2. Utilisation d'un mélange d'alcools aliphatiques primaires supérieurs isolé de poix de tallôl pour la fabrication d'une formulation alimentaire destinée à la réduction des taux de cholestérol sérique chez des sujets humains, **caractérisée en ce que** le mélange d'alcools aliphatiques primaires supérieurs est inclus dans la formulation alimentaire et comprend la composition quantitative suivante en pourcentages en poids :
| | |
|---|---|
| eicosanol | 1,0 - 5,0 |
| docosanol | 5,0 - 30,0 |
| tétracosanol | 20,0 - 60,0 |
| hexacosanol | 15,0 - 50,0 |

3. Formulation alimentaire selon la revendication 1 ou la revendication 2, **caractérisée en ce qu'**elle comprend, de plus, de la margarine de table, du beurre, de la mayonnaise, une huile comestible, du lait, du chocolat, du fromage, de la crème glacée ou du yaourt.

4. Utilisation d'un mélange d'alcools aliphatiques primaires supérieurs isolé de savon de tallôl pour la fabrication d'un médicament destiné à la réduction des taux de cholestérol sérique chez des sujets humains, **caractérisée en ce que** le mélange d'alcools aliphatiques primaires supérieurs est inclus dans le médicament et comprend la composition quantitative suivante en pourcentages en poids :
| | |
|---|---|
| octadécanol | 1,0 - 10,0 |
| eicosanol | 5,0 - 25,0 |
| docosanol | 2,0 - 60,0 |
| tétracosanol | 20,0 - 50,0 |
| hexacosanol | 1,0 - 5,0. |

5. Utilisation d'un mélange d'alcools aliphatiques primaires supérieurs isolé de poix de tallöl pour la fabrication d'un médicament destiné à la réduction des taux de cholestérol sérique chez des sujets humains, **caractérisée en ce que** le mélange d'alcools aliphatiques primaires supérieurs est inclus dans le médicament et comprend la composition quantitative suivante en pourcentages en poids :
| | |
|---|---|
| eicosanol | 1,0 - 5,0 |
| docosanol | 5,0 - 30,0 |
| tétracosanol | 20,0 - 60,0 |
| hexacosanol | 15,0 - 50,0. |

6. Utilisation selon la revendication 4 ou la revendication 5, **caractérisée en ce que** le médicament comprend, de plus, un excipient, un liant, un stabilisant, un lubrifiant, un conservateur ou un agent d'enrobage pharmaceutiquement acceptables.

7. Utilisation selon la revendication 6, **caractérisée en ce que** le médicament est sous la forme de comprimés, capsules, pilules ou sirop.
